# EUROPEAN PATENT SPECIFICATION

(11) **EP 3 011 938 B1**
(45) Date of publication and mention of the grant of the patent: **22.03.2017**
(21) Application number: 15185817.2
(22) Date of filing: 18.09.2015
(51) Int. Cl.: A61F 2/966

(54) **PUSHER GUIDE WIRE**
SCHIEBERFÜHRUNGSDRAHT
GUIDE-FIL A POUSSOIR

(30) Priority: 08.10.2014 JP 2014207392
(43) Date of publication of application: 27.04.2016
(73) Proprietor: PENTAS Inc., Tokyo, 150-0002 (JP)
(72) Inventor: NISHIGISHI, Makoto, Nagoya-shi, Aichi 463-0024 (JP)
(74) Representative: TBK

(56) References cited:
- EP-A1- 2 777 647
- EP-A1- 2 777 648
- US-A1- 2008 300 667
- US-A1- 2009 270 974
- US-A1- 2014 180 387

## Description

### BACKGROUND OF THE INVENTION

### 1. Field of the Invention

The present disclosure relates to a pusher guide wire for delivering a stent housed in a catheter to a target site.
Prior art which is related to this field is disclosed in document US 2008/0300667 A1 showing a system for delivering a stent.

### 2. Description of the Related Art

A stent is a piece of medical equipment used when treating a stricture or a disorder that has developed in a blood vessel or a digestive organ. For example, a stent is used in a case in which a lumen of a blood vessel or a digestive organ is supported in order to prevent the blood vessel or the digestive organ that has been expanded by a balloon catheter from becoming strictured once more, and is used in a case in which an embolus coil in an aneurysm formed in an arterial blood vessel of the abdomen or the brain is confined so that the aneurysm does not become ruptured.

A stent is roughly classified into a balloon-expandable stent that is expanded by a balloon catheter and a self-expandable stent in which the stent itself expands spontaneously. In recent years, self-expandable stents that do not easily become deformed when receiving external force are greatly used.

As a method of delivering a self-expandable stent to a target site, a method is known in which the stent is released to the target site from a distal end of the catheter by pushing a pusher guide wire towards the distal end direction while the stent is held by a stent holding portion of the pusher guide wire (see Japanese Patent No. 4498709 and Japanese Unexamined Patent Application Publication (Translation of PCT Application) No. 2013-521022, for example).

However, in conventional pusher guide wires, the frictional resistance between the stent and the stent holding portion are the same on the distal end side and the proximal end side. Accordingly, even when a technician that has started to release the stent to the target site from the distal end of the catheter notices that the delivering position of the stent is displaced from the target site and pulls the pusher guide wire in the proximal end direction, because the frictional resistance between the stent and the stent holding portion is small, the stent disadvantageously cannot be retrieved into the catheter once more (in other words, the procedure cannot be redone). Accordingly, when delivering the stent to the target site from the distal end of the catheter, the technician needs to perform manipulation in a cautious manner.

### SUMMARY OF THE INVENTION

The inventions is disclosed in the claims. The present disclosure has been made in view of the above circumstances and aims to provide a pusher guide wire that is capable of retrieving a stent, which is in the course of being released from a distal end of a catheter, into the catheter once more by setting the frictional resistance between the stent and a stent holding portion to be larger on a proximal end side than on a distal end side.

The above problem is overcome by the following equipment.

An aspect of the present disclosure is a pusher guide wire for delivering a stent to a target site including a core shaft, a coil body that covers a distal end portion of the core shaft, a pusher portion that is fixed to the core shaft and on a proximal end side with respect to the coil body, and a stent holding portion that is fixed to the core shaft and between the coil body and the pusher portion, in which frictional resistance between the stent and the stent holding portion is larger on the proximal end side than on the distal end side.

In the pusher guide wire of the aspect of the present disclosure, the frictional resistance between the stent and the stent holding portion is larger on the proximal end side than on the distal end side. Since the frictional resistance between the stent and the stent holding portion is smaller on the distal end side, risk of the stent being caught by the stent holding portion can be reduced when the stent is released to the target site from the distal end of the catheter, and since the frictional resistance between the stent and the stent holding portion is larger on the proximal end side, when the technician notices that the delivering position of the stent is displaced from the target site and pulls the pusher guide wire in the proximal end direction, the stent in the course of being released can be retrieved into the catheter (in other words, the procedure can be redone). Furthermore, since the frictional resistance between the stent and the stent holding portion is smaller on the distal end side, when the stent is retrieved into the catheter, risk of the stent coming into contact with the stent holding portion and becoming deformed can be reduced; accordingly, the technician can repeat retrieving the stent into the catheter until the delivery position of the stent coincides with the target site.

### BRIEF DESCRIPTION OF THE DRAWINGS

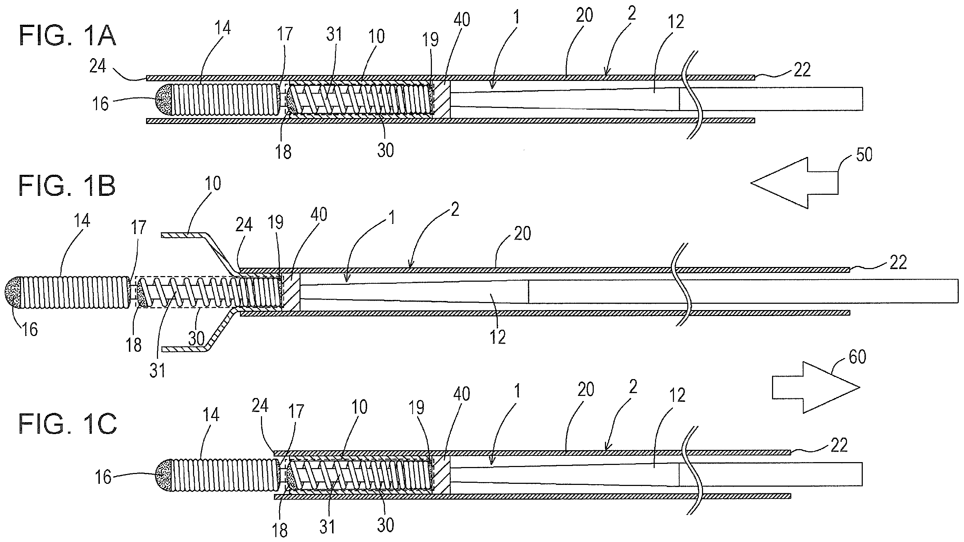

FIGS. 1A to 1C are diagrams each illustrating an entirety of a pusher guide wire of a first exemplary embodiment. FIG. 1A is a diagram illustrating a state in which a stent is housed in a catheter. FIG. 1B is a diagram illustrating a state in which the pusher guide wire is pushed in a distal end direction such that the stent is in the course of being released from a distal end of the catheter. FIG. 1C is a diagram illustrating a state in which the pusher guide wire is pulled in a proximal end direction such that the stent, which is in the course of being released, is retrieved into the catheter.
FIGS. 2A to 2C are diagrams each illustrating an entirety of a pusher guide wire of a second exemplary embodiment. FIGS. 2A to 2C correspond to FIGS. 1A to 1C, respectively.
FIGS. 3A to 3C are diagrams each illustrating an entirety of a pusher guide wire of a third exemplary embodiment. FIGS. 3A to 3C correspond to FIGS. 1A to 1C, respectively.
FIGS. 4A to 4C are useful examples for understanding the invention each illustrating an entirety of a pusher guide wire of a fourth exemplary embodiment.
FIGS. 5A to 5C are each a first modification of FIGS. 4A to 4C, respectively.
FIGS. 6A to 6C are each a second modification of FIGS. 4A to 4C, respectively.
FIGS. 7A to 7C are diagrams each illustrating an entirety of a pusher guide wire of a fifth exemplary embodiment. FIGS. 7A to 7C correspond to FIGS. 1A to 1C, respectively.

### DESCRIPTION OF THE PREFERRED EMBODIMENTS

Referring first to FIGS. 1A to 1C, a pusher guide wire 1 of a first exemplary embodiment will be described.

In FIGS. 1A to 1C, the left side of each drawing is a distal end side (a distal side) of a catheter which is inserted into the body and the right side of each drawing is a proximal end side (a proximal side or a base end side) of the catheter on which manipulation is carried out by a technician, such as a doctor.

As illustrated in FIGS. 1A to 1C, the pusher guide wire 1 for delivering a stent 10 to a target site includes a core shaft 12, a coil body 14 that covers a distal end portion of the core shaft 12, a securing portion 16 that joins the distal end of the core shaft 12 and the distal end of the distal end coil body 14 to each other, a pusher portion 40 that is disposed on the proximal end side (the right side in the drawing) with respect to the coil body 14 and that is fixed to the core shaft 12, and a stent holding portion 30 that is disposed between the coil body 14 and the pusher portion 40 and that is formed of a second coil body 31 that is fixed to the core shaft 12.

The proximal end of the coil body 14 is joined to the core shaft 12 with a securing portion 17. Furthermore, the distal end of the second coil body 31 is joined to the core shaft 12 with a securing portion 18, and the proximal end of the second coil body 31 is joined to the core shaft 12 with a securing portion 19.

A catheter 2 includes a cylindrical body 20 into which the pusher guide wire 1 can be inserted. The pusher guide wire 1 that holds the stent 10 on the outer periphery of the stent holding portion 30 is inserted through a proximal end opening 22 of the catheter 2, and by pushing the core shaft 12 in a distal end direction (the direction of arrow 50), the stent 10 can be released to the target site through the distal end opening 24 of the catheter 2. Note that since a known catheter can be used, the description of the catheter 2 is omitted.

Referring to FIGS. 1A to 1C, a method of delivering the stent 10 that is housed in the catheter 2 to the target site with the pusher guide wire 1 will be described next.

The technician inserts the catheter 2 so that the distal end opening 24 of the catheter 2 coincides with the target site. While the catheter 2 is fixed so as not to move, the pusher guide wire 1 is inserted through the proximal end opening 22 of the catheter 2 and the core shaft 12 is pushed in the distal end direction (the direction of arrow 50) so that the stent 10 is housed in the vicinity of the distal end opening 24 of the catheter 2 (see FIG. 1A). After checking again whether the distal end opening 24 of the catheter 2 coincides with the target site, the core shaft 12 is further pushed in the distal end direction (the direction of arrow 50) such that the stent 10 is released to the target site through the distal end opening 24 of the catheter 2 (see FIG. 1B).

However, in a case in which the distal end portion of the catheter 2 is bent because the target site is located in a bent peripheral blood vessel, when the core shaft 12 is pushed hard in the distal end direction (the direction of arrow 50), there are cases in which the distal end opening 24 of the catheter 2 disadvantageously moves in the distal end direction (the direction of arrow 50) such that the release position of the stent 10 is disadvantageously displaced from the target site. Furthermore, in the course of releasing the stent 10 through the distal end opening 24 of the catheter 2, there may be a case in which one notices that the release position of the stent 10 is displaced from the target site. Furthermore, when the stent 10 is released through the distal end opening 24 of the catheter 2, the stent 10 is expanded in the radial direction and, at the same time, becomes shorter in the axial direction. Accordingly, when the lumen of the blood vessel or the digestive organ is larger than assumed, there are cases in which the release position of the stent 10 is displaced from the target site. In such cases, the stent 10 that is in the course of being released needs to be retrieved into the catheter 2 and the procedure needs to be redone.

As illustrated in FIGS. 1A to 1C, in the pusher guide wire 1 of the present exemplary embodiment, the stent holding portion 30 is formed of the second coil body 31 and the pitch intervals of the second coil body 31 on the proximal end side are smaller than those on the distal end side. With the above, the frictional resistance between the stent 10 and the stent holding portion 30 on the proximal end side is larger than that on the distal end side. Since the frictional resistance between the stent 10 and the stent holding portion 30 is larger on the proximal end side, when noticing that the delivery position of the stent 10 is displaced from the target site, the technician can retrieve the stent 10, which is in the course of being released, into the catheter 2 by pulling the core shaft 12 in a proximal end direction (a direction of arrow 60), and can redo the procedure once again (see FIG. 1C).

Furthermore, in the pusher guide wire 1, since the frictional resistance between the stent 10 and the stent holding portion 30 is smaller on the distal end side, when releasing the stent 10 to the target site through the distal end opening 24 of the catheter 2, risk of the stent 10 being caught by the stent holding portion 30 can be reduced. Furthermore, since the frictional resistance between the stent 10 and the stent holding portion 30 is smaller on the distal end side, as illustrated in FIG. 1C, when the stent 10 is retrieved into the catheter 2, risk of the stent 10 coming into contact with the stent holding portion 30 and becoming deformed can be reduced; accordingly, the technician can repeat retrieving the stent 10 into the catheter 2 until the delivery position of the stent 10 coincides with the target site.

As above, with a simple configuration in which the pitch intervals of the second coil body 31 is changed, the pusher guide wire 1 enables the stent 10 that is in the course of being released to be retrieved into the catheter 2 and the procedure to be redone again (see FIG. 1C).

The materials of the elements configuring the pusher guide wire 1 of the present exemplary embodiment will be described; however, the materials are not particularly limited to the materials described.

The core shaft 12 may be formed of stainless steel (SUS 304, SUS316, or the like) or a super-elastic alloy, such as a Ni-Ti alloy.

The coil body 14 and the second coil body 31 may each be formed of a radiopaque wire. The above may include, for example, gold, platinum, tungsten, or an alloy formed of these elements. By forming the coil body 14 and the second coil body 31 with radiopaque wires, the technician can perceive the positions of the coil body 14 and the second coil body 31 in a fluoroscopic image and, as a result, can perceive the position of the stent 10 housed in the cylindrical body 20 of the catheter 2.

Note that the coil body 14 and the second coil body 31 may each be formed of single wire or may be formed of twisted wire that is a plurality of wires twisted together. Compared with a single wire, the twisted wire is superior in properties such as flexibility and restorability; accordingly, the coil body 14 and the second coil body 31 are desirably formed of twisted wire.

The securing portions 16, 17, 18, and 19 may be formed of a brazing material (aluminum alloy solder, silver solder, gold solder, or the like) or a metal solder (Au-Sn alloy or the like).

Similar to the core shaft 12, the pusher portion 40 may be formed of stainless steel (SUS 304, SUS316, or the like) or a super-elastic alloy, such as a Ni-Ti alloy.

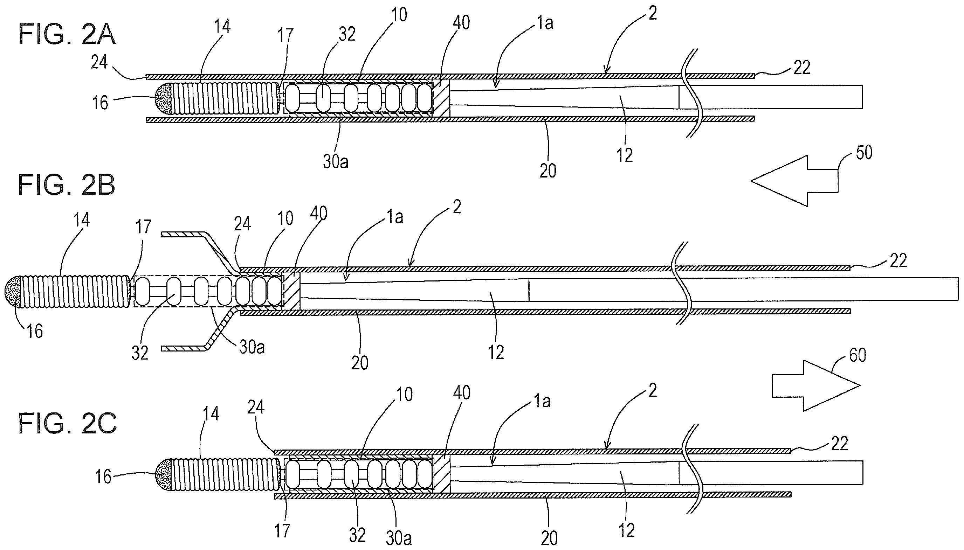

Referring to FIGS. 2A to 2C, a pusher guide wire 1a of a second exemplary embodiment will be described next. Note that similar to FIGS. 1A to 1C, in FIGS. 2A to 2C, the left side of each drawing is a distal end side (a distal side) of the catheter which is inserted into the body and the right side of each drawing is a proximal end side (a proximal side or a base end side) of the catheter on which manipulation is carried out by a technician, such as a doctor.

Points that are different to the pusher guide wire 1 illustrated in FIGS. 1A to 1C will be described. In the pusher guide wire 1a, a stent holding portion 30a is formed of a plurality of bulging portions 32 and the intervals between the bulging portions 32 are smaller on the proximal end side than on the distal end side. With the above, the frictional resistance between the stent 10 and the stent holding portion 30a is larger on the proximal end side than on the distal end side. By setting the intervals between the bulging portions 32 on the distal end side large, the frictional resistance between the stent 10 and the plurality of bulging portions 32 can be made small, and by setting the intervals between the bulging portions 32 on the proximal end side small, the frictional resistance between the stent 10 and the plurality of bulging portions 32 can be made large. As a result, when noticing that the delivery position of the stent 10 is displaced from the target site, the technician can retrieve the stent 10, which is in the course of being released, into the catheter 2 by pulling the core shaft 12 in the proximal end direction (the direction of arrow 60), and can redo the procedure once again (see FIG. 2C).

As above, with a simple configuration in which the intervals of the plurality of bulging portions 32 are changed, the pusher guide wire 1a enables the stent 10 that is in the course of being released to be retrieved into the catheter 2 and the procedure to be redone again (see FIG. 2C).

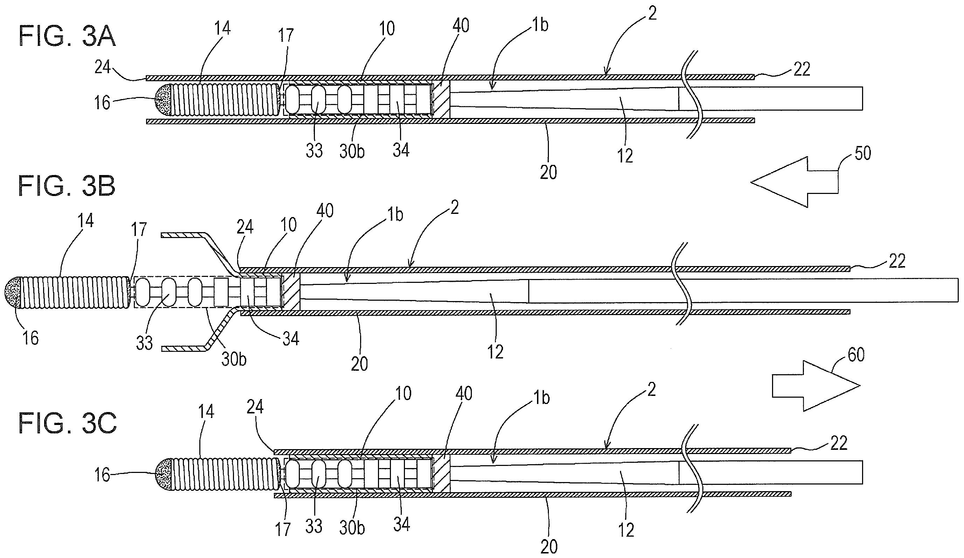

Referring to FIGS. 3A to 3C, a pusher guide wire 1b of a third exemplary embodiment will be described next. Note that similar to FIGS. 1A to 2C, in FIGS. 3A to 3C, the left side of each drawing is a distal end side (a distal side) of the catheter which is inserted into the body and the right side of each drawing is a proximal end side (a proximal side or a base end side) of the catheter on which manipulation is carried out by a technician, such as a doctor.

Points that are different to the pusher guide wire 1a illustrated in FIGS. 2A to 2C will be described. In the pusher guide wire 1b, a plurality of bulging portions 33 are formed on a distal end side of a stent holding portion 30b while a plurality of bulging portions 34 are formed on a proximal end side. Each of the plurality of bulging portions 33 and each of the plurality of bulging portions 34 are fixed to the core shaft 12 at a regular interval. The frictional resistance between the plurality of bulging portions 33 and the stent 10 is small while the frictional resistance between the plurality of bulging portions 34 and the stent 10 is large. With the above, the frictional resistance between the stent 10 and the stent holding portion 30b is larger on the proximal end side than on the distal end side. As a result, when noticing that the delivery position of the stent 10 is displaced from the target site, the technician can retrieve the stent 10, which is in the course of being released, into the catheter 2 by pulling the core shaft 12 in the proximal end direction (the direction of arrow 60), and can redo the procedure once again (see FIG. 3C).

As above, with a configuration that uses the plurality of bulging portions 33 and the plurality of bulging portions 34 that have different shapes, the pusher guide wire 1b enables the stent 10 that is in the course of being released to be retrieved into the catheter 2 and the procedure to be redone again (see FIG. 3C).

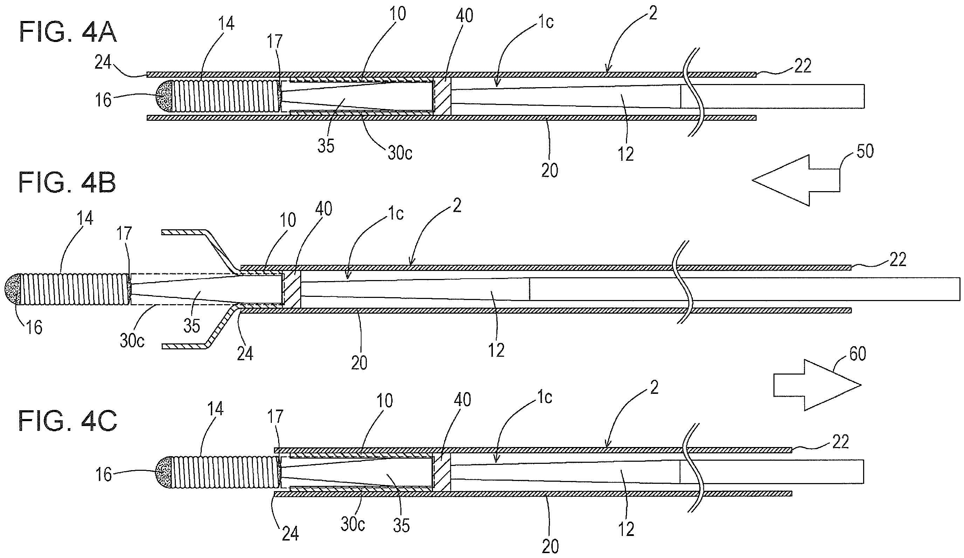

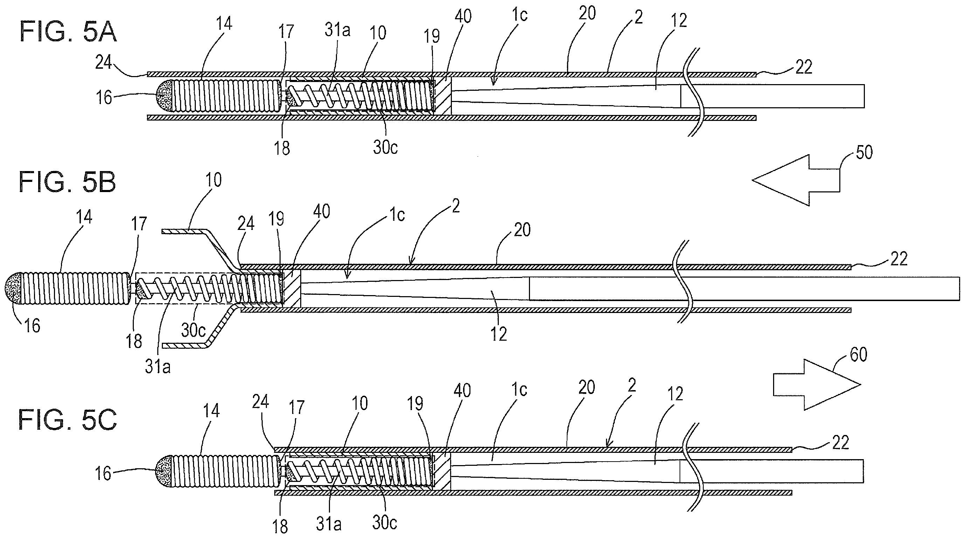

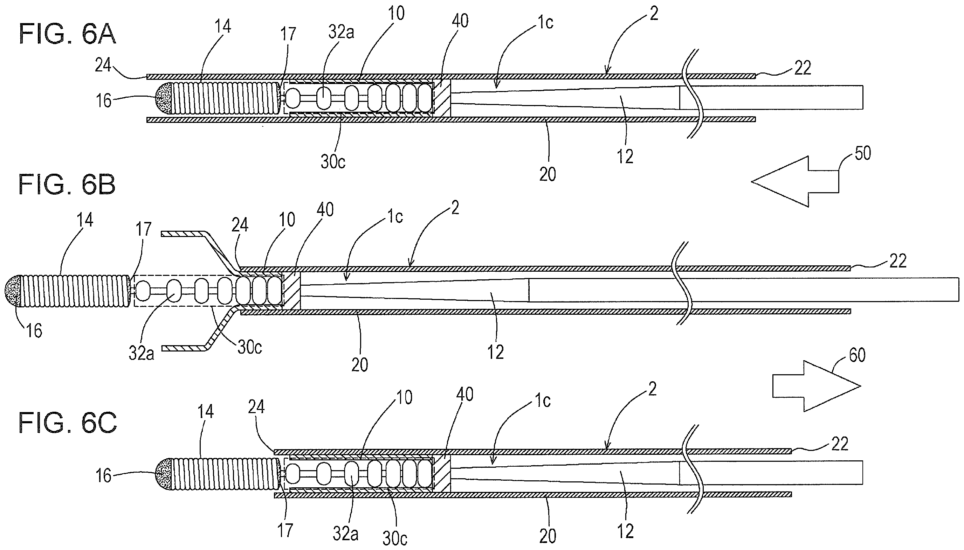

Referring to FIGS. 5A to 6C, a pusher guide wire 1c of a fourth exemplary embodiment will be described next. Note that similar to FIGS. 1A to 3C, in FIGS. 4A to 6C, the left side of each drawing is a distal end side (a distal side) of the catheter which is inserted into the body and the right side of each drawing is a proximal end side (a proximal side or a base end side) of the catheter on which manipulation is carried out by a technician, such as a doctor.

Points that are different to the pusher guide wire 1 illustrated in FIGS. 1A to 1C will be described. In the pusher guide wire 1c illustrated in FIGS. 4A to 4C which are useful examples for understanding the invention, a stent holding portion 30c is formed of a tube body 35 in which the diameter is reduced from the proximal end side towards the distal end side. Accordingly, the gap between the stent 10 and the tube body 35 is smaller on the proximal end side than on the distal end side. With the above, the frictional resistance between the stent 10 and the stent holding portion 30c is larger on the proximal end side than on the distal end side. As a result, when noticing that the delivery position of the stent 10 is displaced from the target site, the technician can retrieve the stent 10, which is in the course of being released, into the catheter 2 by pulling the core shaft 12 in the proximal end direction (the direction of arrow 60), and can redo the procedure once again. Furthermore, when the stent 10 is retrieved into the catheter 2, risk of the stent 10 coming into contact with the stent holding portion 30c and becoming deformed can be further reduced; accordingly, the technician can repeat retrieving the stent 10 into the catheter 2 until the delivery position of the stent 10 coincides with the target site.

As illustrated in FIGS. 5A to 5C, the stent holding portion 30c may be formed of a second coil body 31a in which the diameter is reduced from the proximal end side towards the distal end side. Furthermore, as illustrated in FIGS. 6A to 6C, the stent holding portion 30c may be formed of a plurality of bulging portions 32a whose diameters are reduced from the proximal end side towards the distal end side.

As described above, with a configuration using the tube body 35 whose diameter is reduced from the proximal end side towards the distal end side, the second coil body 31a whose diameter is reduced from the proximal end side towards the distal end side, or the plurality of bulging portions 32a whose diameters are reduced from the proximal end side towards the distal end side, when the stent 10 is retrieved into the catheter 2, the pusher guide wire 1c can reduce the risk of the stent 10 coming into contact with the stent holding portion 30c and becoming deformed (see FIGS. 4C, 5C, and 6C).

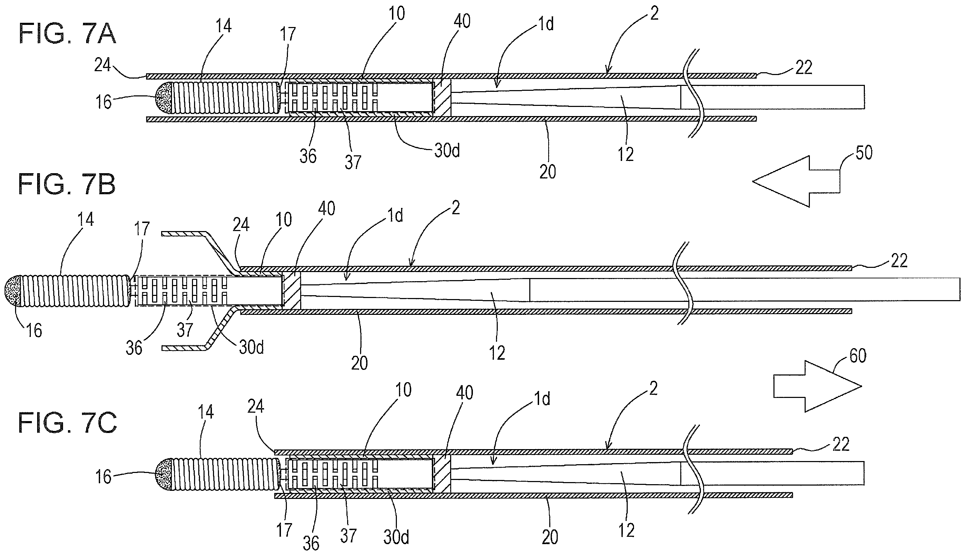

Last of all, referring to FIGS. 7A to 7C, a pusher guide wire 1d of a fifth exemplary embodiment will be described. Note that similar to FIGS. 1A to 6C, in FIGS. 7A to 7C, the left side of each drawing is a distal end side (a distal side) of the catheter which is inserted into the body and the right side of each drawing is a proximal end side (a proximal side or a base end side) of the catheter on which manipulation is carried out by a technician, such as a doctor.

Points that are different to the pusher guide wire 1 illustrated in FIGS. 1A to 1C will be described. In the pusher guide wire 1d, the stent holding portion 30d is provided with a groove portion 36 on the distal end side while a columnar tube body 37 that is provided with no groove portion 36 is formed on the proximal end side. With the above, the frictional resistance between the stent 10 and the stent holding portion 30d is larger on the proximal end side than on the distal end side. As a result, when noticing that the delivery position of the stent 10 is displaced from the target site, the technician can retrieve the stent 10, which is in the course of being released, into the catheter 2 by pulling the core shaft 12 in the proximal end direction (the direction of arrow 60), and can redo the procedure once again (see FIG. 7C).

As described above, in the pusher guide wires 1, 1a, 1b, 1c, and 1d, since the frictional resistance between the stent 10 and the stent holding portions 30, 30a, 30b, 30c, and 30d is larger on the proximal end side, when noticing that the delivery position of the stent 10 is displaced from the target site, the technician can retrieve the stent 10, which is in the course of being released, into the catheter 2 by pulling the core shaft 12 in the proximal end direction (the direction of arrow 60), and can redo the procedure once again. Furthermore, since the frictional resistance between the stent 10 and the stent holding portions 30, 30a, 30b, 30c, and 30d is smaller on the distal end side, when releasing the stent 10 to the target site through the distal end opening 24 of the catheter 2, risk of the stent 10 being caught by the stent holding portions 30, 30a, 30b, 30c, and 30d can be reduced. Furthermore, since the frictional resistance between the stent 10 and the stent holding portions 30, 30a, 30b, 30c, and 30d is smaller on the distal end side, when the stent 10 is retrieved into the catheter 2, risk of the stent 10 coming into contact with the stent holding portions 30, 30a, 30b, 30c, and 30d and becoming deformed can be reduced; accordingly, the technician can repeat retrieving the stent 10 into the catheter 2 until the delivery position of the stent 10 coincides with the target site.

## Claims

1. A pusher guide wire (1, 1c) for delivering a stent (10) to a target site, the pusher guide wire (1, 1c) comprising:
a core shaft (12);
a coil body (14) that covers a distal end portion of the core shaft (12);
a pusher portion (40) that is fixed to the core shaft (12) and on a proximal end side with respect to the coil body (14); and
a stent holding portion (30, 30c) that is fixed to the core shaft (12) and between the coil body (14) and the pusher portion (40), wherein frictional resistance between the stent (10) and the stent holding portion (30, 30c) is larger on the proximal end side than on the distal end side,
**characterized in that**
the stent holding portion (30, 30c) is formed of a second coil body (31, 31a), and pitch intervals of the second coil body (31, 31a) are smaller on the proximal end side than on the distal end side.

2. The pusher guide wire (1c) according to claim 1, wherein
the stent holding portion (30c) is further formed such that diameter of the second coil body (31a) is reduced from the proximal end side towards the distal end side.

3. A pusher guide wire (1a, 1c) for delivering a stent (10) to a target site, the pusher guide wire (1a, 1c) comprising:
a core shaft (12);
a coil body (14) that covers a distal end portion of the core shaft a pusher portion (40) that is fixed to the core shaft (12) and on a proximal end side with respect to the coil body (14); and
a stent holding portion (30a, 30c) that is fixed to the core shaft (12) and between the coil body (14) and the pusher portion (40), wherein frictional resistance between the stent (10) and the stent holding portion (30a, 30c) is larger on the proximal end side than on the distal end side,
**characterized in that**
the stent holding portion (30a, 30c) is formed of a plurality of bulging portions (32, 32a), and intervals between the plurality of bulging portions (32, 32a) are smaller on the proximal end side than on the distal end side.

4. The pusher guide wire (Ic) according to claim 3, wherein
the stent holding portion (30c) is further formed such that diameters of the plurality of the bulging portions (32a) are reduced from the proximal end side towards the distal end side.

5. A pusher guide wire (Ib) for delivering a stent (10) to a target site, the pusher guide wire (Ib) comprising:
a core shaft (12);
a coil body (14) that covers a distal end portion of the core shaft (12);
a pusher portion (40) that is fixed to the core shaft (12) and on a proximal end side with respect to the coil body (14); and
a stent holding portion (30b) that is fixed to the core shaft (12) and between the coil body (14) and the pusher portion (40), wherein frictional resistance between the stent (10) and the stent holding portion (30b) is larger on the proximal end side than on the distal end side,
**characterized in that**
the stent holding portion (30b) is formed of a plurality of first bulging portions (33) and a plurality of second bulging portions (34) that have different shave,
each of the first bulging portions and each of the second bulging portions (33, 34) are fixed to the core shaft (12) at a regular interval,
the friction resistance between the first bulging portion (33) and the stent (10) is smaller than the friction resistance between the second bulging portion (34) and the stent (10), and
the first bulging portions (33) are formed on the distal end side of the stent holding portion (30b), while the second bulging portions (34) are formed on a proximal end side.

6. A pusher guide wire (1d) for delivering a stent (10) to a target site, the pusher guide wire (1d) comprising:
a core shaft (12);
a coil body (14) that covers a distal end portion of the core shaft (12);
a pusher portion (40) that is fixed to the core shaft (12) and on a proximal end side with respect to the coil body (14); and
a stent holding portion (30d) that is fixed to the core shaft (12) and between the coil body (14) and the pusher portion (40), wherein frictional resistance between the stent (10) and the stent holding portion (30d) is larger on the proximal end side than on the distal end side,
**characterized in that**
the stent holding portion (30d) is formed by a columnar tube body (37), and a groove portion (36) is formed on the columnar tube body (37) on the distal end side.

## Patentansprüche

1. Schieberführungsdraht (1, 1c) zum Verbringen einer Gefäßstütze (10) an einen Zielort, mit:
einer Kernwelle (12);
einem Spulenkörper (14), der einen fernen Endabschnitt der Kernwelle (12) bedeckt;
einem Schieberabschnitt (40), der an der Kernwelle (12) und in Bezug auf den Spulenkörper (14) auf einer Seite des nahen Endes fixiert ist, und
einem Gefäßstützehalteabschnitt (30, 30c), der an der Kernwelle (12) und zwischen dem Spulenkörper (14) und dem Schieberabschnitt (40) fixiert ist, wobei der Reibungswiderstand zwischen der Gefäßstütze (10) und dem Gefäßstützehalteabschnitt (30, 30c) auf der Seite des nahen Endes größer als auf der Seite des fernen Endes ist,
**dadurch gekennzeichnet, dass**
der Gefäßstützehalteabschnitt (30, 30c) aus einem zweiten Spulenkörper (31, 31a) ausgebildet ist, und die Zwischenraumintervalle des zweiten Spulenkörpers (31, 31a) auf der Seite des nahen Endes kleiner als auf der Seite des fernen Endes ist.

2. Schieberführungsdraht (1c) nach Anspruch 1, wobei
der Gefäßstützehalteabschnitt (30c) ferner derart ausgebildet ist, dass sich der Durchmesser des zweiten Spulenkörpers (31a) von der Seite des nahen Endes zu der Seite des fernen Endes verringert.

3. Schieberführungsdraht (1a, 1c) zum Verbringen einer Gefäßstütze (10) an einen Zielort, mit:
einer Kernwelle (12);
einem Spulenkörper (14), der einen fernen Endabschnitt der Kernwelle (12) bedeckt;
einem Schieberabschnitt (40), der an der Kernwelle (12) und in Bezug auf den Spulenkörper (14) auf einer Seite des nahen Endes fixiert ist, und
einem Gefäßstützehalteabschnitt (30a, 30c), der an der Kernwelle (12) und zwischen dem Spulenkörper (14) und dem Schieberabschnitt (40) fixiert ist, wobei der Reibungswiderstand zwischen der Gefäßstütze (10) und dem Gefäßstützehalteabschnitt (30a, 30c) auf der Seite des nahen Endes größer als auf der Seite des fernen Endes ist,
**dadurch gekennzeichnet, dass**
der Gefäßstützehalteabschnitt (30a, 30c) aus einer Vielzahl von Wulstabschnitten (32, 32a) ausgebildet ist, und die Intervalle zwischen der Vielzahl von Wulstabschnitten (32, 32a) auf der Seite des nahen Endes kleiner als auf der Seite des fernen Endes sind.

4. Schieberführungsdraht (1c) nach Anspruch 3, wobei
der Gefäßstützehalteabschnitt (30c) ferner derart ausgebildet ist, dass sich die Durchmesser der Vielzahl von Wulstabschnitten (32a) von der Seite des nahen Endes zu der Seite des fernen Endes verringern.

5. Schieberführungsdraht (1b) zum Verbringen einer Gefäßstütze (10) an einen Zielort, mit:
einer Kernwelle (12);
einem Spulenkörper (14), der einen fernen Endabschnitt der Kernwelle (12) bedeckt;
einem Schieberabschnitt (40), der an der Kernwelle (12) und in Bezug auf den Spulenkörper (14) auf einer Seite des nahen Endes fixiert ist, und
einem Gefäßstützehalteabschnitt (30b), der an der Kernwelle (12) und zwischen dem Spulenkörper (14) und dem Schieberabschnitt (40) fixiert ist, wobei der Reibungswiderstand zwischen der Gefäßstütze (10) und dem Gefäßstützehalteabschnitt (30b) auf der Seite des nahen Endes größer als auf der Seite des fernen Endes ist,
**dadurch gekennzeichnet, dass**
der Gefäßstützehalteabschnitt (30b) aus einer Vielzahl von ersten Wulstabschnitten (33) und einer Vielzahl von zweiten Wulstabschnitten (34) mit einer anderen Form ausgebildet ist,
jeder der ersten Wulstabschnitte und jeder der zweiten Wulstabschnitte (33, 34) in regelmäßigen Abständen an der Kernwelle (12) fixiert ist,
der Reibungswiderstand zwischen dem ersten Wulstabschnitt (33) und der Gefäßstütze (10) kleiner als der Reibungswiderstand zwischen dem zweiten Wulstabschnitt (34) und der Gefäßstütze (10) ist, und
die ersten Wulstabschnitte (33) auf der Seite des fernen Endes des Gefäßstützehalteabschnitts (30b) ausgebildet sind, während die zweiten Wulstabschnitte (34) auf der Seite des nahen Endes ausgebildet sind.

6. Schieberführungsdraht (1d) zum Verbringen einer Gefäßstütze (10) an einen Zielort, mit:
einer Kernwelle (12);
einem Spulenkörper (14), der einen fernen Endabschnitt der Kernwelle (12) abdeckt;
einem Schieberabschnitt (40), der an der Kernwelle (12) und in Bezug auf den Spulenkörper (14) auf einer Seite des nahen Endes fixiert ist, und
einem Gefäßstützehalteabschnitt (30d), der an der Kernwelle (12) und zwischen dem Spulenkörper (14) und dem Schieberabschnitt (40) fixiert ist, wobei der Reibungswiderstand zwischen der Gefäßstütze (10) und dem Gefäßstützehalteabschnitt (30d) auf der Seite des nahen Endes größer als auf der Seite des fernen Endes ist,
**dadurch gekennzeichnet, dass**
der Gefäßstützehalteabschnitt (30d) durch einen säulenförmigen Rohrkörper (37) ausgebildet ist, und ein Nutabschnitt (36) auf dem säulenförmigen Rohrkörper (37) auf der Seite des fernen Endes ausgebildet ist.

## Revendications

1. Guide-fil à poussoir (1, 1c) destiné à fournir un stent (10) à un site cible, le guide-fil à poussoir (1, 1c), comprenant :
uu arbre central (12) ;
un corps hélicoïdal (14) qui couvre une partie d'extrémité distale de l'arbre central (12) ;
une partie de poussoir (40) qui est fixée à l'arbre central (12) et sur un côté d'extrémité proximale par rapport au corps hélicoïdal (14) ; et
une partie de retenue de stent (30, 30c) qui est fixée à l'arbre central (12) et entre le corps hélicoïdal (14) et la partie de poussoir (40), la résistance à la friction entre le stent (10) et la partie de retenue de stent (30, 30c) étant plus importante sur le côté d'extrémité proximale que sur le côté d'extrémité distale,
**caractérisé en ce que**
la partie de retenue de stent (30, 30c) est formée par un second corps hélicoïdal (31, 31a) et des intervalles de pas du second corps hélicoïdal (31, 31a) sont plus petits sur le côté d'extrémité proximale que sur le côté d'extrémité distale.

2. Guide-fil à poussoir (1c) selon la revendication 1, dans lequel
la partie de retenue de stent (30c) est en outre formée de sorte que le diamètre du second corps hélicoïdal (31a) diminue du côté d'extrémité proximale vers le côté d'extrémité distale.

3. Guide-fil à poussoir (1a, 1c) destiné à fournir un stent (10) à un site cible, le guide-fil à poussoir (1a, 1c) comprenant :
un arbre central (12) ;
un corps hélicoïdal (14) qui couvre une partie d'extrémité distale de l'arbre central
une partie de poussoir (40) qui est fixée à l'arbre central (12) et sur un côté d'extrémité proximale par rapport au corps hélicoïdal (14) ; et
une partie de retenue de stent (30a, 30c) qui est fixée à l'arbre central (12) et entre le corps hélicoïdal (14) et la partie à poussoir (40), la résistance à la friction entre le stent (10) et la partie de retenue de stent (30a, 30c) étant plus importante sur le côté d'extrémité proximale que sur le côté d'extrémité distale,
**caractérisé en ce que**
la partie de retenue de stent (30a, 30c) est formée par une pluralité de parties protubérantes (32, 32a) et des intervalles entre la pluralité des parties protubérantes (32, 32a) sont plus petits sur le côté d'extrémité proximale que sur le côté d'extrémité distale.

4. Guide-fil à poussoir (1c) selon la revendication 3, dans lequel
la partie de retenue de stent (30c) est en outre formée de sorte que les diamètres de la pluralité des parties protubérantes (32a) diminuent du côté d'extrémité proximale vers le côté d'extrémité distale.

5. Guide-fil à poussoir (1b) destiné à fournir un stent (10) à un site cible, le guide-fil à poussoir (1b) comprenant :
un arbre central (12) ;
un corps hélicoïdal (14) qui couvre une partie d'extrémité distale de l'arbre central (12) ;
une partie de poussoir (40) qui est fixée à l'arbre central (12) et sur un côté d'extrémité proximale par rapport au corps hélicoïdal (14) ; et
une partie de retenue de stent (30b) qui est fixée à l'arbre central (12) et entre le corps hélicoïdal (14) et la partie de poussoir (40), la résistance à la friction entre le stent (10) et la partie de retenue de stent (30b) étant plus importante sur le côté d'extrémité proximale que sur le côté d'extrémité distale,
**caractérisé en ce que**
la partie de retenue de stent (30b) est formée par une pluralité de premières parties protubérantes (33) et une pluralité de secondes parties protubérantes (34) qui ont une forme différente.
chacune des premières parties protubérantes et chacune des secondes parties protubérantes (33, 34) est fixée à l'arbre central (12) selon un intervalle régulier,
la résistance à la friction entre la première partie protubérante (33) et le stent (10) étant plus petite que la résistance à la friction entre la seconde partie protubérante (34) et le stent (10), et
les premières parties protubérantes (33) sont formées sur le côté d'extrémité distale de la partie de retenue de stent (30b), tandis que les secondes parties protubérantes (34) sont formées sur un côté d'extrémité proximale.

6. Guide-fil à poussoir (1d) destiné à fournir un stent (10) à un site cible, le guide-fil à poussoir (1d), comprenant :
un arbre central (12) ;
un corps hélicoïdal (14) qui couvre une partie d'extrémité distale de l'arbre central (12) ;
une partie de poussoir (40) qui est fixée à l'arbre central (12) et sur un côté d'extrémité proximale par rapport au corps hélicoïdal (14) ; et
une partie de retenue de stent (30d) qui est fixée à l'arbre central (12) et entre le corps hélicoïdal (14) et la partie de poussoir (40), la résistance à la friction entre le stent (10) et la partie de retenue de stent (30d) étant plus importante sur le côté d'extrémité proximale que sur le côté d'extrémité distale,
**caractérisé en ce que**
la partie de retenue de stent (30d) est formée par un corps tubulaire en forme de colonne (37) et une partie de renfoncement (36) est formée sur le corps tubulaire en forme de colonne (37) sur le côté d'extrémité distale.
